(19) Europäisches Patentamt
European Patent Office
Office européen des brevets

(11) Veröffentlichungsnummer: **0 004 535**
**B1**

(12) **EUROPÄISCHE PATENTSCHRIFT**

(45) Veröffentlichungstag der Patentschrift: **29.04.81**

(51) Int. Cl.$^3$: **C 07 D 233/64**

(21) Anmeldenummer: **79100213.2**

(22) Anmeldetag: **25.01.79**

# (54) Verfahren zur Herstellung von 4-Methyl-5-((2-aminoäthyl)-thiomethyl)-imidazol-dihydrochlorid.

(30) Priorität: **03.04.78 DE 2814355**

(43) Veröffentlichungstag der Anmeldung:
**17.10.79 Patentblatt 79/21**

(45) Bekanntmachung des Hinweises auf die Patenterteilung:
**29.04.81 Patentblatt 81/17**

(84) Benannte Vertragsstaaten:
**CH DE FR GB IT**

(56) Entgegenhaltungen:
**LU - A - 77 140**
**US - A - 3 950 353**
**US - A - 4 055 573**

(73) Patentinhaber: **BASF Aktiengesellschaft**
**Carl-Bosch-Strasse 38**
**D-6700 Ludwigshafen (DE)**

(72) Erfinder: **Halbritter, Klaus**
**Schwarzwaldstrasse 19**
**D-6800 Mannheim 1 (DE)**

**EP 0 004 535 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

Verfahren zur Herstellung von 4-Methyl-5-[(2-aminoäthyl)-thiomethyl]-imidazol-dihydrochlorid

Die Erfindung betrifft ein einstufiges Verfahren zur Herstellung von 4-Methyl-5-[(2-aminoäthyl)-thiomethyl]-imidazol-dihydrochlorid durch Thiomethylierung von 4-Methylimidazol.

Es ist bekannt, daß man das 4-Methyl-5-[(2-aminoäthyl)-thiomethyl]-imidazol-dihydrochlorid aus dem 4-Methyl-5-hydroxymethyl-imidazol-hydrochlorid durch Umsetzung mit Cysteaminhydrochlorid in siedender Essigsäure oder siedender konzentrierter Salzsäure, wie es beispielsweise in der DE—A—22 11 454 beschrieben wird, herstellen kann. Nachteilig an diesem Verfahren ist, daß von 4-Methyl-5-hydroxymethyl-imidazol ausgegangen wird, da diese Verbindung nur verhältnismäßig umständlich durch Reduktion von 4-Methyl-imidazol-5-carbonsäureestern mit Lithiumaluminiumhyrid (J. Med. Chem. *19*, 923—928 (1976)) oder mit Alkalimetallen oder Calcium in flüssigem Ammoniak (DE—A—26 37 670) hergestellt werden kann. Weiterhin ist ein zweistufiges Herstellungsverfahren vorgeschlagen worden EP—A—0 003 052, nach dem man 4-Methyl-5-chlormethylimidazol-hydrochlorid mit Cysteamin-hydrochlorid umsetzt.

Es wurde nun gefunden, daß man 4-Methyl-5-[(2-aminoäthyl)-thiomethyl]-imidazol-dihydrochlorid in einem einstufigen Verfahren herstellen kann, indem man 4-Methylimidazol in überschüssiger wäßriger konzentrierter Salzsäure mit Cysteamin und Formaldehyd oder einem Oligomeren des Formaldehyds oder mit Thiazolidin oder Bis-(N-thiazolidinyl)-methan in einem geschlossenen System bei Temperaturen von 110 bis 170°C umsetzt.

Das erfindungsgemäße Verfahren kann beispielsweise durch das folgende Formelschema veranschaulicht werden:

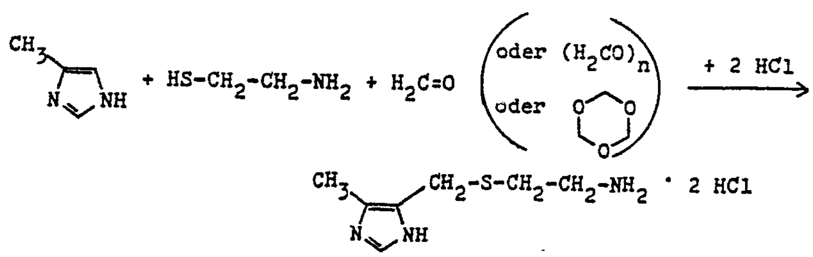


Bei dem erfindungsgemäßen Verfahren werden zweckmäßigerweise das 4-Methylimidazol, Cysteamin und der Formaldehyd oder eines seiner Oligomeren in wäßriger konzentrierter Salzsäure gelöst, wobei in der Regel 20- bis 40 gew.%-ige Lösungen in Bezug auf das Gewicht der Ausgangsverbindungen hergestellt werden. Die Ausgangsverbindungen 4-Methylimidazol, Cysteamin und Formaldehyd, bezogen auf monomeren Formaldehyd, werden vorteilhaft in einem Molverhältnis von 0,9—1,1: 0,9—1,1: 0,9—1,4 verwendet. Diese Molverhältnisse gelten auch bei der Verwendung von Thiazolidin oder Bis-(N-thiazolidinyl)-methan, die bekannterweise als 1:1- bzw. 2:3-Kondensationsprodukte von Cysteamin und Formaldehyd aufgefaßt werden können.

Das 4-Methylimidazol kann dabei als Reinsubstanz oder in Form der üblichen technischen Qualität, ca. 92- bis 98%-ig, eingesetzt werden. Das Cysteamin wird als Base oder als Hydrochlorid verwendet.

Der Formaldehyd wird entweder gasförmig oder in üblicher wäßriger Lösung, enthaltend 30 bis 40 Gew.% Formaldehyd, als Paraformaldehyd oder als s-Trioxan verwendet.

In einer besonderen Ausgestaltung des erfindungsgemäßen Verfahrens werden die Kondensationsprodukte von Cysteamin und Formaldehyd, nämlich Thiazolidin oder Bis-(N-thiazolidinyl)-methan oder ihre Hydrochloride, mit 4-Methylimidazol unter den erfindungsgemäßen Bedingungen umgesetzt. Dabei kann es bei der Umsetzung von Thiazolidin vorteilhaft sein, wenn eine zusätzliche Menge Formaldehyd oder eines seiner Oligomeren, etwa eine Menge von 5 bis 20, besonders bevorzugt etwa 10 Mol%, berechnet auf das Thiazolidin, dem reaktionsgemisch zugefügt wird.

Der Chlorwasserstoff wird in überschüssiger Menge, vorteilhafterweise als wäßrige konzentrierte Salzsäure, enthaltend 30 bis 40 Gew.% Chlorwasserstoff, verwendet. Dabei ist in der Regel die molare Konzentration an Chlorwasserstoff 3-bis 8-mal so hoch wie die des 4-Methylimidazols. Im Falle der Verwendung von waßriger Formaldehydlösung kann daher gegebenenfalls zusätzlich HCl-Gas bis zur gewünschten Konzentration in die Reaktionslösung eingeleitet werden.

Die erfindungsgemäße Thiomethylierungsreaktion wird in einem geschlossenen System, beispielsweise in einem Emaillekessel oder Tantalautoklaven, bei Temperaturen von 110 bis 170°C, vorzugsweise von 115 bis 145°C, durchgeführt. Gemäß den angewandten Temperaturen stellt sich in dem geschlossenen System der entsprechende Druck ein, der in der Regel etwa zwischen 0,5 bis 12 bar, und entsprechend dem bevorzugten Temperaturbereich bei 3,5 bis 5,5 bar liegt.

Die Umsetzung ist im allgemeinen innerhalb eines Zeitraumes von 5 bis 30 Stunden beendet. Der Verlauf der Umsetzung kann ohne Schwierigkeiten spektroskopisch verfolgt werden, beispielsweise indem man Proben nach Behandlung mit $D_2O$ NMR-spektroskopisch analysiert.

Die Aufarbeitung des Reaktionsproduktes erfolgt in an sich üblicher Weise, beispielsweise durch Abdestillieren des Lösungsmittels und Umkristallisieren z.B. in einem niederen Alkohol mit 1 bis 4 C-Atomen, wie Äthanol oder n-Propanol, oder einer niederen Carbonsäure, wie Essigsäure. Gegebenenfalls kann statt des Umkristallisierens auch mit den genannten Lösungsmitteln digeriert werden. Gewünschtenfalls kann nach üblichen Methoden ohne weiteres auch die freie Base hergestellt werden.

Das erfindungsgemäße einstufige Verfahren zeichnet sich durch überraschende Einfachheit aus. Es war nicht zu erwarten, daß die Reaktion in der gefundenen Weise abläuft. Alkylthiomethylierungen mit Cysteamin als Thiol sind nicht literaturbekannt. Allgemein sind für Alkylthiomethylierungen nur wenige Beispiele in der Literatur beschrieben, wie es beispielsweise aus J. Mathieu und J. Weill-Raynal in Formation of C—C-Bonds, Vol. 1, Georg Thieme Verlag, Stuttgart 1973, hervorgeht. Diese Beispiele beschränken sich ausschließlich auf reaktive carbocyclische Aromaten. für Heterocyclen, wie Imidazole, ist diese Reaktion bisher noch nicht beschrieben worden. Für den Fachmann war daher eine Thiomethylierung des 4-Methylimidazols mit Mercaptoäthylamin (Cysteamin) nicht vorhersehbar. Vielmehr war die bekannte Reaktion des Formldehyds mit Cysteaminhydrochlorid zum Thiazolidinhydrochlorid zu erwarten:

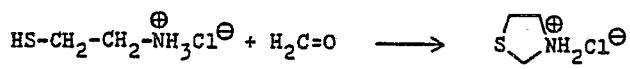

Diese Thiazolidinbildung verläuft bekannterweise unter sehr milden Bedingungen bei Raumtemperaturen, vgl. S. Ratner und H. T. Clarke in J. Amer. Chem. Soc. *59*, 200—206 (1937). Das Thiazolidin bildet sich sogar in hoher Ausbeute, wie aus dem Vergleichsbeispiel 6 hervorgeht, durch Umsetzung von Cysteamin mit Paraformaldehyd in siedender wäßriger konzentrierter Salzsäure in Gegenwart von 4-Methylimidazol.

Im Hinblick auf das 4-Methylimidazol ist insbesondere hervorzuheben, daß die Thiomethylierung mit hoher Selektivität in 5-Stellung und nicht in 2-Stellung erfolgt, auch werden mögliche Aminomethylierungsreaktionen als Nebenreaktionen nicht beobachtet.

Von besonderer praktischer Bedeutung ist die Tatsache, daß bei dem erfindungsgemäßen Verfahren die theoretisch mögliche Bildung von Bis-chlormethyläther nicht beobachtet wird.

Die Erfindung macht das 4-Methyl-5-[(2-aminoäthyl)-thiomethyl]-imidazol-dihydrochlorid, das ein wichtiges Zwischenprodukt für weitere Imidazolderivate darstellt, technisch leichter als bisher zugänglich.

Das 4-Methyl-5-[(2-aminoäthyl)-thiomethyl]-imidazol ist ein wichtiges Zwischenprodukt beispielsweise für die Herstellung des Arzneimittels Cimetidin (N-Cyan-N'-methyl-N''-[2-((4-methyl-5-imidazolyl)-methylmercapto)-äthyl]-guanidin), wie es in den deutschen Offenlegungsschriften 23 44 779 und 26 49 059 beschrieben wird.

Das erfindungsgemäße Verfahren soll durch die folgenden Beispiele beschrieben, aber nicht eingeschränkt werden.

Beispiel 1

42,0 Teile 97,5%-iges 4-Methylimidazol, 56,8 Teile Cysteamin-hydrochlorid und 18,0 Teile Paraformaldehyd werden unter Kühlung in 207 Teilen 37%-iger wäßriger Salzsäure gelöst, so daß die Temperatur nicht über 30°C steigt. In einem geschlossenen Emaillekessel wird das Gemisch 5 Stunden auf 110 bis 120°C und weitere 10 Stunden auf 120°C erhitzt. Danach wird bei maximal 80°C im Wasserstrahlvakuum die Salzsäure bis fast zur Trockne abdestilliert und der Rückstand in 237 Teilen siedendem Äthanol gelöst. Es wird auf 20°C abgekühlt und der ausgefallene Niederschlag abfiltriert und getrocknet. Man erhält 81,9 Teile (1. Fraktion) 4-Methyl-5-[(2-aminoäthyl)-thiomethyl]-imidazol-dihydrochlorid vom Schmp. 179 bis 180°C.

Das Filtrat wird etwa auf das halbe Volumen eingedampft, man kühlt aur 20°C ab und filtriert den Niederschlag ab. Nach dem Trocknen erhält man weitere 12,7 Teile vom Schmp. 149 bis 154°C (2. Fraktion).

Die Fraktionen 1 und 2 werden vereinigt und aus 315 Teilen Eisessig umkristallisiert. Nach dem Trocknen bei 10 Torr und 90°C erhält man 82,4 Teile (67,5%) 4-Methyl-5-[(2-aminoäthyl)-thiomethyl]-imidazol-dihydrochlorid vom Schmp. 190 bis 191°c.

Beispiel 2

44,3 Teile 92,5%-iges 4-Methylimidazol, 38,6 Teile Cysteamin und 17,7 Teile Paraformaldehyd werden in 260 Teilen 37%-iger wäßriger Salzsäure gelöst und es wird in einem geschlossenen Emaillekessel 20 Stunden auf 140°C erhitzt. Bei maximal 80°C wird anschließend im Wasserstrahlvakuum die Salzsäure bis fast zur Trockne abdestilliert und der Rückstand in 356 Teilen siedendem Äthanol gelöst. Nach dem Abkühlen auf 20°C wird der Niederschlag abfiltriert und getrocknet. Man erhält 74 Teile (61%) vom Schmp. 188 bis 190°C.

Das Filtrat wird etwa zur Hälfte eingeengt und auf 5°C gekühlt. Es werden nach dem Abfiltrieren und Trocknen weitere 12,3 Teile (10%) vom Schmp. 181 bis 185°C erhalten.

Statt der Kristallisation in zwei Fraktionen kann man das bis fast zur Trockne abdestillierte

Reaktionsgemisch mit 472 Volumenteilen n-Propanol (wobei sich Volumenteile zu Gewichtsteile verhalten wie Liter zu Kilogramm) 1 Stunde in der Siedehitze diegerieren, anschließend noch in der Hitze abfiltrieren und den Rückstand trocknen. Man erhält 89,6 Gewichsteile, entsprechend 73,5% Ausbeute, fast farblose Kristalle vom Schmp. 191,4 bis 193,6°C.

Beispiel 3

27 Teile 97%-iges 4-Methylimidazol, 37,5 Teile Cysteamin-hydrochlorid und 10,8 Teile Paraformaldehyd werden in 124 Teilen 37%iger wäßriger Salzsäure gelöst und das Gemisch wird in einem geschlossenen Tantalautoklaven 10 Stunden auf 150°C erhitzt. Anschließend wird bei maximal 80°C im Wasserstrahlvakuum die Salzsäure bis fast zur Trockne abdestilliert und der Rückstand in 237 Teilen siedendem Äthanol gelöst. Nach dem Abkühlen auf 20°C wird der Niederschlag abfiltriert, mit 50 Teilen eiskaltem Äthanol gewaschen und getrocknet. Man erhält 53 Teile (68%) vom Schmp. 186 bis 188°C.

Beispiel 4

18,8 Teile 93%-iges 4-Methylimidazol, 25,1 Teile Thiazolidin-hydrochlorid und 0,9 Teile Paraformaldehyd werden in 95 Teilen 37%-iger Salzsäure in einem geschlossenen Emaillekessel 10 Stunden auf 130°C erhitzt.

Die Salzsäure wird anschließend bei maximal 80°C bis fast zur Trockne abdestilliert und der Rückstand in 237 Teilen siedendem Äthanol gelöst. Nach dem Abkühlen auf 20°C wird der Niederschlag abfiltriert, mit 40 Teilen kaltem Äthanol gewaschen und getrocknet. Man erhält 26 Teile (53%) vom Schmp. 188 bis 189°C. Durch Aufarbeiten der Mutterlauge und anschließender Umkristallisation in Eisessig werden weitere 8 Teile (16%) vom Schmp. 188 bis 189°C erhalten.

Beispiel 5

28,5 Teile Bis-(N-thiazolidinyl)-methan und 26,5 Teile 93%-iges 4-Methylimidazol werden in 124 Teilen 37%-iger wäßriger Salzsäure gelöst und in einem geschlossenen Emaillekessel 10 Stunden auf 130°C erhitzt. Anschließend wird bei maximal 80°C die Salzsäure bis fast zur Trockne abdestilliert und der Rückstand in 237 Teilen siedendem Äthanol nahezu gelöst. Nach dem Abkühlen auf 20°C wird abfiltriert, mit 40 Teilen Äthanol gewaschen und getrocknet. Man erhält 41,2 Teile (56%) vom Schmp. 183 bis 185°C.

Durch Einengen des Filtrats auf etwa 2/5 des ursprünglichen Volumens erhält man nach dem Abfiltrieren und Trocken weitere 7 Teile (10%) vom Schmp. 181 bis 184°C.

Beispiel 6

Vergleichsbeispiel unter Normaldruck:

Eine Lösung von 8,8 Teilen 93%-iges 4-Methylimidazol, 7,7 Teilen Cysteamin und 3,0 Teilen Paraformaldehyd in 47,5 Teilen 37%-iger wäßriger Salzsäure wird 1 Stunde zum Sieden erhitzt (106°C). Anschließend wird im Wasserstrahlvakuum stark eingeengt, mit 16 Teilen Äthanol versetzt und zum Sieden erhitzt. Nach dem Abkühlen auf 5°C werden die ausgefallenen Kristalle abgesaugt, mit 40 Teilen kaltem Äthanol gewaschen und getrocknet.

Man erhält 8,2 Teile (65%) Thiazolidin-hydrochlorid vom Schmp. 169 bis 171°C. Die analysenreine Verbindung, umkristallisiert aus Äthanol, hat einem Schmelzpunkt von 180°C.

Die NMR-spektroskopische Umsetzung zeigt, daß das Rohprodukt ca. 3 Gew.% 4-Methyl-5-[(2-aminoäthyl)-thiomethyl]-imidazol-dihydrochlorid enthält.

**Patentansprüche**

1. Verfahren zur Herstellung von 4-Methyl-5-[(2-aminoäthyl)-thiomethyl]-imidazol-dihydrochlorid, dadurch gekennzeichnet, daß man 4-Methylimidazol in überschüssiger wäßriger konzentrierter Salzsäure mit Cysteamin und Formaldehyd oder einem Oligomeren des Formaldehyds oder mit Thiazolidin oder Bis-(N-thiazolidinyl)-methan in einem geschlossenen System bei Temperaturen von 110 bis 170°C umsetzt.

2. Verfahren nach Anspruch 1, dadurch gekennzeichnet, daß man 4-Methylimidazol, Cysteamin und Formaldehyd in Molverhältnissen von 0,9—1,1: 0,9—1,1: 0,9—1,4 umsetzt.

**Revendications**

1. Procédé de préparation de dihydrochlorure de 4-méthyl-5-[(2-aminoéthyl)-thiométhyl]-imidazole, caractérisé par le fait qu'on fait reagir en système ferme à des températures de 110 à 170°C, du 4-méthylimidazole dans de l'acide chlorhydrique aqueux concentré en excès, avec de la cystéamine et du formaldéhyde ou un oligomère de formaldéhyde ou avec de la thiazolidine ou du bis-(N-thiazolidinyl)-méthane.

2. Procédé selon la revendication 1, dans lequel on fait réagir du 4-méthylimidazole, de la cystéamine et du formaldéhyde en proportion molaire de 0,9—1,1: 0,9—1,1: 0,9—1,4.

## Claims

1. A process for the preparation of 4-methyl-5-[(2-aminoethyl)-thiomethyl]-imidazole dihydrochloride, characterized in that 4-methylimidazole, in excess aqueous concentrated hydrochloric acid, is reacted with cysteamine and formaldehyde or a formaldehyde oligomer, or with thiazolidine or bis-(N-thiazolidinyl)-methane, in a closed system, at from 110 to 170°C.

2. A process as claimed in claim 1, characterized in that 4-methylimidazole, cysteamine and formaldehyde are reacted in a molar ratio of 0.9—1.1: 0.9—1.1: 0.9—1.4.